# EUROPÄISCHE PATENTSCHRIFT

(11) **EP 0 515 828 B1**
(45) Veröffentlichungstag und Bekanntmachung des Hinweises auf die Patenterteilung: **22.05.1996**
(21) Anmeldenummer: 92106749.2
(22) Anmeldetag: 21.04.1992
(51) Int. Cl.: A61B 17/56

(54) **Trochanterstabilisierungsvorrichtung**
Trochanter stabilisation device
Dispositif de stabilisation du trochanter

(30) Priorität: 30.05.1991 CH 1602/91
(43) Veröffentlichungstag der Anmeldung: 02.12.1992
(73) Patentinhaber: Synthes AG, Chur, CH-7002 Chur (CH)
(72) Erfinder: Weigum, Hans, CH-4435 Niederdorf (CH)
(74) Vertreter: Lusuardi, Werther Giovanni, Dr.

(56) Entgegenhaltungen:
- EP-A- 0 347 874
- WO-A-81/02388
- FR-A- 1 093 696
- FR-A- 2 149 943
- US-A- 4 973 332

## Beschreibung

Die Erfindung bezieht sich auf eine Vorrichtung gemäss der Gattung des Patentanspruchs 1.

Solche Vorrichtungen werden insbesondere zur Versorgung von Schenkelhalsbrüchen mit zusätzlichen Trochanterfrakturen des Femur eingesetzt.

Eine solche Vorrichtung ist aus der EP-A2 0 347 874 bekannt, welche ebenfalls eine konventionelle Hülsenlasche und eine damit lösbar verbindbare Trochanterstabilisierungsplatte umfasst. Nachteilig bei dieser bekannten zweiteiligen Vorrichtung ist jedoch deren ineinandergreifende Konstruktion, welche dazu zwingt die Trochanterstabilisierungsplatte direkt am Knochen zu befestigen und die Hülsenlasche über der am Knochen anliegenden Trochanterstabilisierungsplatte anzubringen. Bei dieser zwingenden Reihenfolge der beiden Elemente sollte der Chirurg sich von allem Anfang an für oder gegen den Einsatz einer Trochanterstabilisierungsplatte entscheiden. Ein nachträglicher Einbau einer Trochanterstabilisierungsplatte - nach erfolgter Versorgung eines Knochenbruches mit einer Hülsenlasche - ist nicht mehr möglich ohne die Hülsenlasche wieder vollständig zu entfernen. In der klinischen Praxis ist es wünschenswert, wenn der Entscheid, zusätzlich eine Trochanterstabilisierungsplatte zu verwenden, möglichst spät gefällt werden kann.

Aus der WO/81/02388 ist eine Vorrichtung zum Fixieren des Oberschenkelhalses am Schaft eines Oberschenkelknochens bekannt, welche aus einem in den Oberschenkelhals einschlagbaren Nagel und einer am grossen Rollhügel des Oberschenkelknochens fixierbaren Krallenplatte besteht. Beide Elemente sind gegeneinander verspannt und mit separaten Schrauben am Knochen befestigt. Diese Vorrichtung ist somit völlig starr und in keiner Weise anpassbar.

Aus der US 4,973,332 ist schliesslich eine Hülsenlasche bekannt, welche mit einer zusätzlichen Platte am Femur befestigt wird. Die zusätzliche Platte erstreckt sich jedoch nur bis unterhalb des Trochanters, so dass dieser in keiner Weise von der Vorrichtung gestützt wird.

Hier will die Erfindung Abhilfe schaffen. Der Erfindung liegt die Aufgabe zugrunde, eine leicht montierbare, universell je nach den jeweiligen Bedürfnissen anwendbare, fertigungstechnisch einfache Vorrichtung zum Verbinden eines, insbesondere im Bereich des Femurhalses gebrochenen Knochens zu schaffen.

Die Erfindung löst die gestellte Aufgabe mit einer Vorrichtung, welche die Merkmale des Anspruchs 1 aufweist, sowie einer Trochanterstabilisierungsplatte, welche die Merkmale des Anspruchs 10 aufweist.

Die durch die Erfindung erreichten Vorteile sind im wesentlichen darin zu sehen, dass dank der erfindungsgemässen Vorrichtung auch eine nachträgliche Montage der Trochanterstabilisierungsplatte auf eine bereits eingebaute Hülsenlasche ermöglicht wird. Um den festen Sitz einer bereits montierten Hülsenlasche nicht wieder lockern zu müssen, ist mindestens eine Befestigungsperforation der Schaftpartie der Trochanterstabilisierungsplatte für das darin einzuführende Befestigungselement, vorzugsweise eine Knochenbefestigungsschraube, vollständig durchgängig ausgebildet. Vorteilhafterweise wird dazu dasjenige Schraubenloch gewählt, welches der in den Trochanter einzuführenden Knochenbefestigungsschraube am zweitnächsten gelegen ist, es kann jedoch auch eines der übrigen Schraubenlöcher gewählt werden. Die Durchgängigkeit des einen Schraubenlochs für den Kopf der Knochenbefestigungsschraube macht, bei einer nachträglichen Montage der Trochanterstabilisierungsplatte, die Lockerung der Hülsenlasche unnötig.

Um die Trochanterstabilisierungsplatte gegen Verbiegung in der medio-lateralen Ebene zu versteifen und um gleichzeitig eine Verdrehung der Trochanterstabilisierungsplatte gegenüber der Hülsenlasche zu verhindern sind die Kanten der longitudinalen Schaftpartie mindestens in einem Teilbereich seitlich abgebogen, sodass eine Schiene resultiert, welche auf die am Knochen anliegende Knochenbefestigungslasche der Hülsenlasche aufgedrückt, bzw. aufgeschnappt werden kann. Durch den dadurch erreichten festen Sitz kann eine Reibungskorrosion - wie sie bei solchen Kombinationsvorrichtungen auftreten können - vermieden werden.

Bei einer bevorzugten Ausführungsform der Erfindung sind die longitudinale Schaftpartie der Trochanterstabilisierungsplatte und die Knochenbefestigungslasche der Hülsenlasche hohlzylindersektorförmig ausgebildet um eine möglichst gute Anpassung an die Anatomie des annährend kreiszylindrischen Röhrenknochens zu erreichen. Diese hohlzylindrische Ausgestaltung der Trochanterstabilisierungsplatte und der Knochenbefestigungslasche verbessert zudem die Biegesteifigkeit dieser Elemente.

Vorteilhafterweise ist die als äussere Schiene für die Knochenbefestigungslasche der Hülsenlasche ausgebildete Trochanterstabilisierungsplatte als elastische Klammer konstruiert, welche lediglich mit den Enden ihrer Kanten die Knochenbefestigungslasche der Hülsenlasche federnd umschliesst. Diese federnde Umklammerung gestaltet die sonst kritische Passung der beiden ineinandergreifenden Teile weniger kritisch.
Im weiteren sind die Kanten der longitudinalen Schaftpartie der Trochanterstabilisierungsplatte um einen Winkel α zwischen 1° und 5°, vorzugsweise zwischen 2° und 4°, gegenüber den Laschenkanten abgebogen, was einen grösseren Radius als bei der entsprechenden seitlichen Laschenkante an der Hülsenlasche ermöglicht. Dies hat werkstoff- und verarbeitungstechnische Vorteile, da sich dadurch das für die Trochanterstabilisierungsplatte verwendete Blech hoher Festigkeit mit einem grösseren Radius und damit mit einer geringeren spezifischen Dehnung gebogen werden kann. Es ergeben sich aber auch Vorteile konstruktiver Art, insbesondere bei toleranzmässig kritischen Fällen mit einer engen Passung bleibt bei abgebogenen Kanten der Flankenberührungspunkt an der gleichen Stelle, was einen maximalen Aufbiege-Hebelarm und damit eine minimale Aufbiegekraft ermöglicht; die Kantenabbiegung bewirkt somit eine verbesserte Dimensionstoleranz der erfindungsgemässen Vorrichtung.

Bei einer weiteren bevorzugten Ausführungsform der Erfindung sind die korrespondierenden Befestigungsperforationen der Knochenbefestigungslasche mit einer Ansenkung und die dazu korrespondierenden Befestigungsperforationen der Schaftpartie mit einer in die Ansenkung eingreifenden entsprechenden Vertiefung versehen. Durch dieses formschlüssige Eingreifen der beiden Platten wird eine axiale Verschiebung der beiden Platten gegeneinander verhindert. Dieser Effekt wird durch die in die korrespondierenden Befestigungsperforationen eingeführten Befestigungsmittel noch verstärkt. Eine absolut starre Verbindung der beiden Platten ist zur Vermeidung von Reibkorrosion äusserst wichtig. Zudem ergibt sich dadurch fertigungstechnisch eine geringere Bauhöhe. Auch die Schraubenköpfe überragen die kombinierte erfindungsgemässe Vorrichtung nur um etwa die Blechstärke. Bei einer guten Anschmiegung der genannten Ansenkungen und Vertiefungen bleibt die Möglichkeit einer dynamischen Kompression erhalten.

Das Anbringen von vorzugsweise in longitudinaler Richtung verlaufenden Versteifungssicken im Bereich der Trochanterabstützpartie, welche sich bis in den Bereich der Schaftpartie erstrecken können, verstärkt die Steifigkeit der Trochanterstabilisierungsplatte weiter. Um eine über die gesamte Länge der Trochanterstabilisierungsplatte gleichmässige Steifigkeit zu erreichen, sollten sich die Sicken und die abgebogenen Kanten überlappen. Das ausgebogene, bzw. angesenkte Loch (ev. in Form eines Langloches) für die unmittelbar oberhalb der Knochenbefestigungsschraube einzuführende Antirotationsschraube hat ebenfalls einen zusätzlichen versteifenden Effekt, der alternativ oder komplementär zu den Sicken wirken kann.

Im Gegensatz zu gattungsmässig gleichen Vorrichtungen gemäss dem Stand der Technik, bei welchen die Kontaktflächen zwischen Hülsenlasche und Trochanterstabilisierungsplatte aufeinander abgestimmt sein müssen, genügt bei der erfindungsgemässen Vorrichtung im angegebenen Bereich eine einheitliche Länge der Trochanterstabilisierungsplatte, beispielsweise mit 4 Befestigungsperforationen, unabhängig davon wie lange die entsprechende Partie der verwendeten Hülsenlasche ist. Auch für die Trochanterabstützpartie, insbesondere wenn diese adaptierbar gestaltet ist, genügt eine Länge und eine Form, so dass es möglich ist eine einzige standardisierte Trochanterstabilisierungsplatte für sämtliche Anwendungen zu benützen.

Diese Vorteile zusammen mit der möglichen Herstellung aus Blech erlaubt eine äusserst kostengünstige Fertigung bei hoher Stückzahl. Dadurch, dass die Steifigkeit der Trochanterstabilisierungsplatte durch deren Wölbung, die umgebogenen Kanten und vorzugsweise durch deren Sicken erzielt wird, kann bei der Herstellung ein relativ dünnes Blech verwendet werden. Dies wiederum ermöglicht es, dass die Trochanterabstützpartie intraoperativ zur Erzielung einer besseren Passung mit dem Trochanter vom Chirurgen zurecht gebogen werden kann.

Ein Ausführungsbeispiel der Erfindung, welches zugleich das Funktionsprinzip erläutert, ist in der Zeichnung dargestellt und wird im folgenden näher beschrieben.

Es zeigen
Fig. 1 eine perspektivische Ansicht der erfindungsgemässen Vorrichtung;
Fig. 2 eine Ansicht von vorne der Trochanterstabilisierungsplatte gemäss Fig. 1;
Fig. 3 eine Ansicht von der Seite der Trochanterstabilisierungsplatte gemäss Fig. 1;
Fig. 4 eine Ansicht von hinten der Trochanterstabilisierungsplatte gemäss Fig. 1;
Fig. 5 eine Seitenansicht der, mit der als Schiene wirkenden Trochanterstabilisierungsplatte zusammengesteckten Hülsenlasche;
Fig. 6 einen Längsschnitt durch die zusammengesteckte, am Femur angebrachte Vorrichtung gemäss Fig. 5;
Fig. 7 einen Querschnitt längs der Linie VII-VII durch die Vorrichtung gemäss Fig. 5;
Fig. 8 einen Querschnitt längs der Linie VIII-VIII durch die Vorrichtung gemäss Fig. 5; und
Fig. 9 einen Querschnitt längs der Linie IX-IX durch die Trochanterstabilisierungsplatte gemäss Fig. 4.

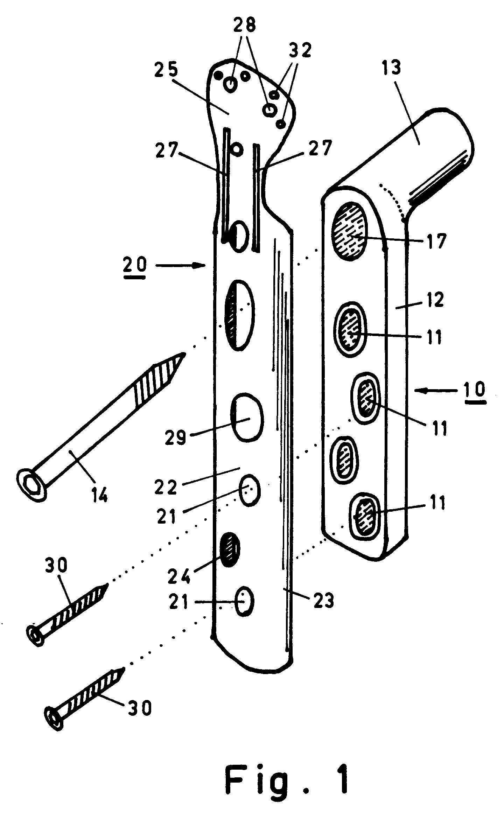

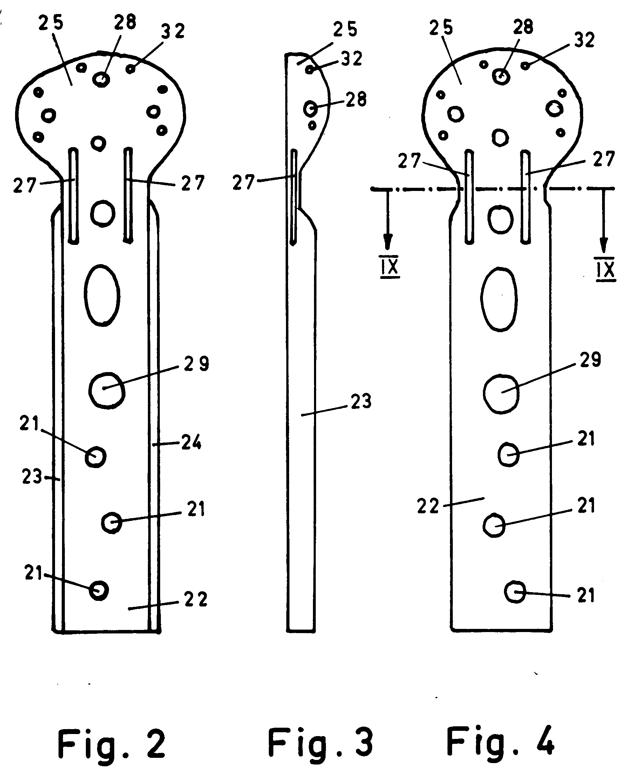

Gemäss den Fig. 1 - 5 besteht die erfindungsgemässe Vorrichtung im wesentlichen aus einer konventionellen Hülsenlasche 10, wie sie zur Versorgung von Schenkelhalsbrüchen des Femur verwendet werden, sowie einer Trochanterstabilisierungsplatte 20.

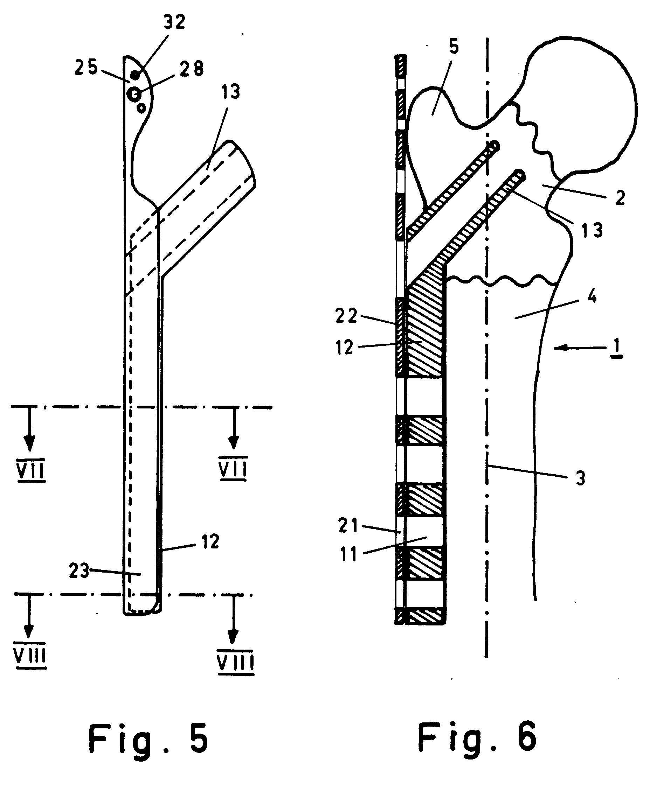

Die konventionelle Operationstechnik zur Implantation der Hülsenlasche 10 besteht darin, dass man mittels geeigneter Zielgeräte eine Bohrung in latero-medialer Richtung von unterhalb des Trochanters 5 durch das Zentrum des Schenkelhalses 2 setzt, in welche dann die Hülse 13 der Hülsenlasche 10 eingeführt werden kann. Schliesslich wird die Hülsenlasche 10 mit ihrer Lasche 12 am Femurschaft 4 verschraubt (diese Situation ist in Fig. 6 dargestellt).

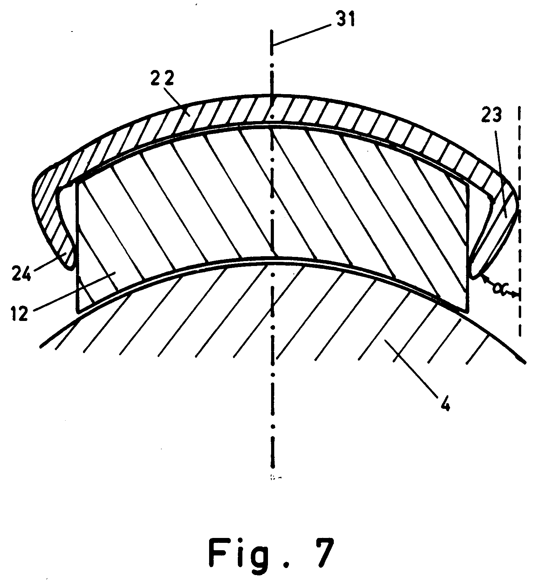

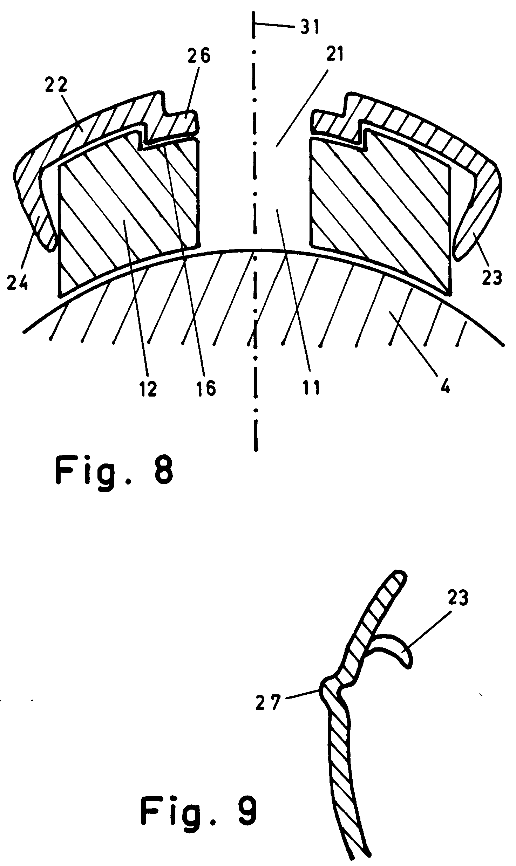

Zu diesem Zweck besteht die Hülsenlasche 10 aus einer mit dem Femurschaft 4 verbindbaren, parallel zur Längsachse 3 des Femurschaftes 4 verlaufenden, mit einer Anzahl Befestigungsperforationen 11 versehenen Knochenbefestigungslasche 12 und einer daran in einem Winkel stehenden Hülse 13 mit einer Bohrung 17, durch welche die Knochenschraube 14, bzw. ein funktionsmässig entsprechender Knochennagel, hindurchgeführt werden kann. Die Befestigungsperforationen 11 sind vorzugsweise versetzt angeordnet und wie in Fig. 8 dargestellt mit einer Ansenkung 16 versehen. Zur Fixation der Knochenbefestigungslasche 12 werden konventionelle Befestigungsschrauben 30 verwendet. Zur besseren anatomischen Adaptation an die gewölbte Knochenoberfläche ist die Knochenbefestigungslasche 12 der Hülsenlasche 10 als daran adaptierter Hohlzylindersektor ausgebildet (Fig. 7 und 8).

Das zweite, lösbar mit der Hülsenlasche 10 verbindbare Element der erfindungsgemässen Vorrichtung besteht aus einer Trochanterstabilisierungsplatte 20, welche im wesentlichen in eine longitudinale Schaftpartie 22 und eine daran anschliessende Trochanterabstützpartie 25 gegliedert ist.
Die parallel zur Knochenbefestigungslasche 12 der Hülsenlasche 10 verlaufende, longitudinalen Schaftpartie 22 ist mit einer Anzahl Befestigungsperforationen 21 versehen, welche mit den Befestigungsperforationen 11 der Knochenbefestigungslasche 12 fluchten. Zur Erzielung eines stabilen Verbundes der Trochanterstabilisierungsplatte 20 mit der Hülsenlasche 10 sind die Befestigungsperforationen 21 der Schaftpartie 22 mit einer in die Ansenkung 16 der Befestigungsperforationen 11 eingreifenden, entsprechenden Vertiefung 26 versehen.

Die Schaftpartie 22 weist eine äussere Seite und eine innere, zum Knochen, bzw. zur am Knochen angebrachten Hülsenlasche gerichtete Seite auf und ist, gleich wie die Knochenbefestigungslasche 12 der Hülsenlasche 10, als Hohlzylindersektor ausgebildet (Fig. 7 und 8). Die seitlichen Kanten 23 und 24 der Schaftpartie 22 sind in Richtung zur inneren Seite abgebogen, so dass eine äussere Schiene für die am Knochen anliegende Knochenbefestigungslasche 12 der Hülsenlasche 10 resultiert. Die Kanten 23 und 24 schliessen mit der Schaftpartie 22 - wie in Fig. 7 gezeigt - einen Winkel α von beispielsweise 3° ein (relativ zur Symmetriebene 31), so dass eine elastische Klammer zur federnden Aufnahme der Knochenbefestigungslasche 12 der Hülsenlasche 10 resultiert.

An die kaudal gelegene Schaftpartie 22 schliesst eine nach kranial gerichtete Trochanterabstützpartie 25 an, welche körbchenartig geformt und mit einer Anzahl Befestigungsperforationen 28 mit einem Durchmesser von etwa 4 mm versehen ist. Zusätzlich zu den Befestigungsperforationen 28 ist dem Rand entlang eine Reihe von kleineren Perforationen 32 von etwa 3 mm Durchmesser vorgesehen, welche zur alternativen, bzw. komplementären Verwendung von Cerclagedraht dienen. Die grösseren Befestigungsperformationen 28 eigenen sich dazu nicht, weil sie zu diesem Zweck ungünstig liegen und durch die Ansenkungen zwangsläufig scharfkantige Ränder aufweisen, die den Cerclagedraht durchschneiden könnten.

Wie in Fig. 9 dargestellt, sind zur Versteifung des Überganges zwischen Schaftpartie 22 und Trochanterabstützpartie 25 zwei longitudinale Sicken 27 vorgesehen, welche sich bis in den Bereich der Schaftpartie 22 erstrecken.

Die Knochenbefestigungslasche 12 kann somit in die als äussere Schiene gestaltete Schaftpartie 22 der Trochanterstabilisierungsplatte 20 eingeführt und die miteinander korrespondierenden Befestigungsperforationen 11 und 21 zur Deckung gebracht werden, so dass mittels der als Befestigungsmittel dienenden Fixationsschrauben 30 beide Elemente der erfindungsgemässen Vorrichtung gemeinsam mit dem Knochen 1 verbindbar sind. Die Trochanterabstützpartie ist deformierbar ausgebildet, so dass sie intraoperativ der Trochantergeometrie angepasst werden kann und mittels geeigneter (nicht dargestellter), in die Befestigungsperforationen 28 einzuführender Schrauben an den Trochanter 5 befestigt werden kann.

Dadurch, dass eine der Befestigungsperforationen 29 der Schaftpartie 22 für das darin einzuführende Befestigungsmittel 30 vollständig durchgängig ausgebildet ist, d.h. gegenüber den übrigen Befestigungsperforationen 21 grösser ist, kann die Trochanterstabilisierungsplatte 20 auch noch nachträglich auf eine bereits am Knochen fixierte Hülsenlasche 10 montiert werden, ohne letztere wieder lockern zu müssen.

Die Trochanterstabilisierungsplatte 20 kann dank ihrer konstruktiven Steifigkeit aus einem ziemlich dünnen Blech gefertigt werden. Als Material eignet sich vorzugsweise rostfreier Stahl.

## Patentansprüche

1. Vorrichtung zum Verbinden eines im Gelenkbereich (2) gebrochenen Knochens (1), bestehend aus
einer Hülsenlasche (10) mit
einer mit dem Knochen (1) verbindbaren, parallel zur Längsachse (3) des Knochens (1) verlaufenden, mit Befestigungsperforationen (11) versehenen Knochenbefestigungslasche (12) und
einer daran in einem Winkel stehenden Hülse (13) zur Aufnahme eines in den Gelenkbereich des Knochens (1) einzuführenden Befestigungselementes (14),
einer lösbar mit der Hülsenlasche (10) verbindbaren Trochanterstabilisierungsplatte (20), mit
einer parallel zur Knochenbefestigungslasche (12) verlaufenden, korrespondierende Befestigungsperforationen (21) aufweisende, nach kaudal gerichteten, mindestens in einem Teilbereich seitlich abgebogene Kanten (23,24) aufweisenden, longitudinalen Schaftpartie (22) und
einer an die Schaftpartie (22) anschliessenden nach kranial gerichteten Trochanterabstützpartie (25),
dadurch gekennzeichnet, dass
die longitudinale Schaftpartie (22) der Trochanterstabilisierungsplatte (20) mit den beiden seitlich abgebogenen Kanten (23,24) als äussere Schiene für die am Knochen anliegende Knochenbefestigungslasche (12) der Hülsenlasche (10) ausgebildet ist, und
Trochanterstabilisierungsplatte (20) und Hülsenlasche (10) mittels in die korrespondierenden Befestigungsperforationen (11,21) einführbaren Befestigungsmittel (30) gemeinsam mit dem Knochen (1) verbindbar sind.

2. Vorrichtung nach Anspruch 1, dadurch gekennzeichnet, dass die longitudinale Schaftpartie (22) der Trochanterstabilisierungsplatte (20) und die Knochenbefestigungslasche (12) der Hülsenlasche (10) hohlzylindersektorförmig ausgebildet sind.

3. Vorrichtung nach Anspruch 1 oder 2, dadurch gekennzeichnet, dass die als äussere Schiene für die Knochenbefestigungslasche (12) der Hülsenlasche (10) ausgebildete Trochanterstabilisierungsplatte (20) als elastische Klammer konstruiert ist, welche mit den Enden ihrer Kanten (23,24) die Knochenbefestigungslasche (12) der Hülsenlasche (10) federnd umschliesst.

4. Vorrichtung nach Anspruch 3, dadurch gekennzeichnet, dass die Kanten (23,24) der longitudinalen Schaftpartie (22) der Trochanterstabilisierungsplatte (20) gegenüber der Symmetrieebene (31) der Trochanterstabilisierungsplatte (20) einen Winkel α zwischen 1° und 5°, vorzugsweise zwischen 2° und 4° einschliessen.

5. Vorrichtung nach einem der Ansprüche 1 bis 4, dadurch gekennzeichnet, dass die korrespondierenden Befestigungsperforationen (11) der Knochenbefestigungslasche (12) mit einer Ansenkung (16) versehen sind und die dazu korrespondierenden Befestigungsperforationen (21) der Schaftpartie (22) mit einer in die Ansenkung (16) eingreifenden entsprechenden Vertiefung (26) versehen sind.

6. Vorrichtung nach einem der Ansprüche 1 bis 5, dadurch gekennzeichnet, dass die Trochanterabstützpartie (25) mit vorzugsweise in longitudinaler Richtung verlaufenden Versteifungssicken (27) versehen ist, die sich vorzugsweise bis in den Bereich der Schaftpartie (22) erstrecken.

7. Vorrichtung nach einem der Ansprüche 1 bis 6, dadurch gekennzeichnet, dass mindestens eine der Befestigungsperforationen (21) der Schaftpartie (22) für das darin einzuführende Befestigungsmittel (30) vollständig durchgängig ausgebildet ist.

8. Vorrichtung nach einem der Ansprüche 1 bis 7, dadurch gekennzeichnet, dass die Trochanterstabilisierungsplatte (20) mit Befestigungsperforationen (28) versehen ist.

9. Vorrichtung nach einem der Ansprüche 1 bis 8, dadurch gekennzeichnet, dass die Trochanterstabilisierungsplatte (20) aus einem 1,25 bis 1,85 mm , vorzugsweise 1,35 bis 1,7 mm dünnen Blech gefertigt ist.

10. Trochanterstabilisierungsplatte (20) für eine Vorrichtung nach einem der Ansprüche 1 bis 9, mit
einer parallel zur Knochenbefestigungslasche (12) verlaufenden, korrespondierende Befestigungsperforationen (21) aufweisende, nach kaudal gerichteten, mindestens in einem Teilbereich seitlich abgebogene Kanten (23,24) aufweisenden, longitudinalen Schaftpartie (22) und
einer an die Schaftpartie (22) anschliessenden nach kranial gerichteten Trochanterabstützpartie (25),
wobei die seitlich abgebogenen Kanten (23 , 24) als äussere Schiene für die Knochenbefestigungslasche (12) der Hülsenlasche (10) konstruiert sind und als elastische Klammer federnd die Knochenbefestigungslasche umschliessen.

## Claims

1. Device for treating a bone (1) with a fracture in the joint area (2) comprising
a sleeve strap (10) having
a bone attachment leg (12) running parallel to the longitudinal axis (3) of the bone (1) for application to the bone, said leg (12) having fixation apertures (11), and
a sleeve (13), positioned at an angle to the bone attachment leg (12) for receiving a connecting element (14) for insertion into the joint area (2) of the bone (1),
a trochanter stabilization plate (20) for separable connection with the sleeve strap (10), having
a caudally oriented longitudinal shaft part (22) running parallel to the bone attachment leg (12) and having corresponding fixation apertures (21) and at least in a partial area laterally bent edges (23,24), and
a cranially oriented trochanter support part (25) connected to said longitudinal shaft part (22),
characterized in that
the longitudinal shaft part (22) of the trochanter stabilization plate (20) with the two laterally bent edges (23,24) is designed as external track for the bone attachment leg (12) adjacent to the bone (1) of the sleeve strap (10), and
the trochanter stabilization plate (20) and the sleeve strap (10) are jointly connectable to the bone (1) by means of fixation means (30) introducible into the corresponding fixation apertures (21,11).

2. Device according to claim 1, characterized in that the longitudinal shaft part (22) of the trochanter stabilization plate (20) and the bone attachment leg (12) of the sleeve strap (10) have the shape of a sector of a hollow cylinder.

3. Device according to claim 1 or 2, characterized in that the trochanter stabilization plate (20) serving as external track for the bone attachment leg (12) of the sleeve strap (10) is designed as a flexible clamp which by means of the ends of its edges (23,24) encompasses elastically the sleeve strap (10).

4. Device according to claim 3, characterized in that the edges (23,24) of the longitudinal shaft part (22) of the trochanter stabilization plate (20) form, in relation to the plane of symmetry (31) of the trochanter stabilization plate (20), an angle α of between 1° and 5°, preferably between 2° and 4°.

5. Device according to one of the claims 1 to 4, characterized in that the fixation apertures (11) of the bone attachment leg (12) are provided with countersinkings (16), and that the fixation apertures (21) of the shaft part (22) corresponding therewith are provided with corresponding recesses (26) engaging into the countersinkings (16).

6. Device according to one of the claims 1 to 5, characterized in that the trochanter support part (25) has reinforcement ribs (27) running preferably in the longitudinal direction and which preferably extend to the area of the shaft part (22).

7. Device according to one of the claims 1 to 6, characterized in that at least one of the fixation apertures (21) of the shaft part (22) is enlarged to completely accommodate a fixation means (30) introduced therein.

8. Device according to one of the claims 1 to 7, characterized in that the trochanter stabilization plate (20) has attachment perforations (28).

9. Device according to one of the claims 1 to 8, characterized in that the trochanter stabilization plate (20) is sheet metal between 1.25 and 1.85 mm, preferably between 1.35 and 1.70 mm thick.

10. A trochanter stabilization plate (20) for use with a device according to one of the claims 1 to 9, with
a caudally oriented longitudinal shaft part (22) running parallel to the bone attachment leg (12) and having corresponding fixation apertures (21) and at least in a partial area laterally bent edges (23,24), and
a cranially oriented trochanter support part (25) connected to said longitudinal shaft part (22),
whereby the laterally bent edges (23,24) are designed as external track for the bone attachment leg (12) of the sleeve strap (10), and encompass elastically the bone attachment leg (12) as a flexible clamp.

## Revendications

1. Dispositif pour la liaison d'un os fracturé (1) dans la région de l'articulation (2), constitué de une patte à douille (10), comportant:
une patte de fixation sur l'os (12) pouvant être reliée à l'os (1), s'étendant parallèlement à l'axe longitudinal (3) de l'os (1) et pourvue de perforations de fixation (11), et
une douille oblique (13), pour la réception d'un élément de fixation (14) à insérer dans la région de l'articulation de l'os (1),
une plaque de stabilisation du grand trochanter (20) pouvant être reliée de manière libérable à la patte à douille (10), et comportant
une partie longitudinale en tige (22), s'étendant parallèlement à la patte de fixation sur l'os (12), présentant des perforations de fixation correspondantes (21), tournée dans la direction caudale, et présentant au moins dans une région partielle des bords (23, 24) repliés latéralement, et
une partie de soutien du grand trochanter (25) se raccordant à la partie en tige (22) et orientée dans la direction crânienne,
caractérisé en ce que
la partie longitudinale en tige (22) de la plaque de stabilisation du grand trochanter (20) est configurée avec les deux bords (23, 24) latéraux repliés, sert de rail externe pour la patte de fixation sur l'os (12), reposant sur l'os, de la patte à douille (10), et
la plaque de stabilisation du grand trochanter (20) et la patte à douille (10) peuvent être reliées ensemble à l'os (1) au moyen de moyens de fixation (30) pouvant être insérés dans les perforations de
fixation correspondantes (11, 21).

2. Dispositif selon la revendication 1, caractérisé en ce que la partie longitudinale en tige (22) de la plaque de stabilisation du grand trochanter (20) et la patte de fixation sur l'os (12) de la patte à douille (10) sont configurées en forme de secteur cylindrique creux.

3. Dispositif selon la revendication 1 ou 2, caractérisé en ce que la plaque de stabilisation du grand trochanter (20), configurée comme rail externe pour la patte de fixation sur l'os (12) de la patte à douille (10), est construite comme pince élastique qui, par les extrémités de ses bords (23, 24), entoure à ressort la patte de fixation sur l'os (12) de la patte à douille (10).

4. Dispositif selon la revendication 3, caractérisé en ce que les bords (23, 24) de la partie longitudinale en tige (22) de la plaque de stabilisation du grand trochanter (20) forme avec le plan de symétrie (31) de la plaque de stabilisation du grand trochanter (20) un angle α valant entre 1° et 5°, de préférence entre 2° et 4°.

5. Dispositif selon l'une quelconque des revendications 1 à 4, caractérisé en ce que les perforations de fixation correspondantes (11) de la patte de fixation sur l'os (12) sont pourvues d'un renfoncement (16), et en ce que les perforations de fixation correspondantes (21) de la partie en tige (22) sont pourvues d'un épaississement correspondant (26) s'engageant dans le renfoncement (16).

6. Dispositif selon l'une quelconque des revendications 1 à 5, caractérisé en ce que la partie de soutien du grand trochanter (25) est pourvue de nervures de renforts (27) que s'étendent de préférence dans la direction longitudinale et, de préférence, jusque dans la région de la partie en tige (22).

7. Dispositif selon l'une quelconque des revendications 1 à 6, caractérisé en ce qu'au moins une des perforations de fixation (21) de la partie en tige (22) est configurée de telle sorte que le moyen de fixation (30) qui doit y être inséré puisse la traverser complètement.

8. Dispositif selon l'une quelconque des revendications 1 à 7, caractérisé en ce que la plaque de stabilisation du grand trochanter (20) est pourvue de perforations de fixation (28).

9. Dispositif selon l'une quelconque des revendications 1 à 8, caractérisé en ce que la plaque de stabilisation du grand trochanter (20) est fabriquée à partir d'une tôle mince de 1,25 à 1,85 mm, de préférence de 1,35 à 1,7 mm.

10. Plaque de stabilisation du grand trochanter (20) pour un dispositif selon l'une quelconque des revendications 1 à 9, comportant
une partie longitudinale en tige (22) s'étendant parallèlement à la patte de fixation sur l'os (12), présentant des perforations de fixation correspondantes (21), orientée dans la direction caudale, et présentant au moins dans une région partielle des bords (23, 24) repliés latéralement, et
une partie de soutien du grand trochanter (25) se raccordant à la partie en tige (22) et orientée dans la direction crânienne,
tandis que les bords latéraux (23, 24) repliés sont construits sous la forme d'un rail externe pour la patte de fixation sur l'os (12) de la patte à douille (10) et enferment élastiquement la patte de fixation sur l'os, sous la forme d'une pince élastique.
